(19) Europäisches Patentamt
European Patent Office
Office européen des brevets

(11) Veröffentlichungsnummer: **0 106 089**
**A1**

# (12) EUROPÄISCHE PATENTANMELDUNG

(21) Anmeldenummer: **83108618.6**

(22) Anmeldetag: **01.09.83**

(51) Int. Cl.[3]: **A 61 K 7/13**

(30) Priorität: **10.09.82 DE 3233541**

(43) Veröffentlichungstag der Anmeldung: **25.04.84 Patentblatt 84/17**

(84) Benannte Vertragsstaaten: **AT BE CH DE FR GB IT LI NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Rose, David, Dr.**
**Holbeinweg 7**
**D-4010 Hilden(DE)**

(72) Erfinder: **Lieske, Edgar**
**Hunsrückenstrasse 40**
**D-4000 Düsseldorf(DE)**

(54) **Haarfärbemittel.**

(57) Oxidationshaarfärbemittel enthalten als Oxidationsfarbstoffvorprodukte Resorcinderivate der allgemeinen Formel

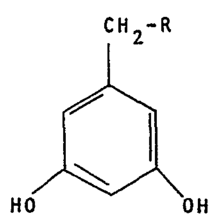

in der R eine $-NH_2$-Gruppe, eine $-OH$-Gruppe oder eine $-COOH$-Gruppe bedeutet oder deren Salze als Kupplerkomponente und übliche Entwicklersubstanzen, bevorzugt aromatische und/oder heterocyclische Diamine oder 2.4.5.6-Tetraaminopyrimidin. Die neuen Haarfärbemittel liefern überwiegend braune Farbnuancen, die gegenüber Licht und Wärme besonders stabil sind.

EP 0 106 089 A1

Henkelstraße 67
4000 Düsseldorf, den 7.9.1982

HENKEL KGaA
ZR-FE/Patente
Dr. JG/Br

P a t e n t a n m e l d u n g

D 6644 EP

# H a a r f ä r b e m i t t e l

Gegenstand der Erfindung sind Haarfärbemittel auf der Basis von Oxidationsfarbstoffen. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt, die unter dem Einfluß von Oxidationsmitteln oder von Luftsauerstoff Farbstoffe ausbilden. Als kosmetische Träger für die Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haaren spielen die sogenannten Oxidationsfarben, die durch oxidative Kupplung einer oder mehrerer Entwicklerkomponenten untereinander oder mit einer oder mehreren Kupplerkomponenten entstehen, wegen ihrer intensiven Farben und guten Echtheitseigenschaften eine bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopyridinderivate, heterocyclische Hydrazonderivate, 4-Aminopyrazolonderivate und Tetraaminopyrimidine eingesetzt. Als sogenannte Kupplersubstanzen werden m-Phenylendiaminderivate, Naphthole, Resorcinderivate und Pyrazolone verwendet.

...

Gute Oxidationshaarfarbstoffvorprodukte müssen in erster Linie folgende Voraussetzungen erfüllen: Sie müssen bei der oxidativen Kupplung die gewünschten Farbnuancen in ausreichender Intensität ausbilden. Sie müssen ferner ein gutes Aufziehvermögen auf menschlichem Haar besitzen ohne die Kopfhaut zu stark anzufärben und sie sollen vor allem in toxikologischer und dermatologischer Hinsicht unbedenklich sein.

Die Verwendung von Resorcin als Kuppler ist aus den deutschen Patentschriften Nr. 162.625 und Nr. 276 761 bekannt. Darüber hinaus sind weitere Resorcinderivate als Kuppler für Oxidationshaarfärbemittel vorgeschlagen worden, zum Beispiel Mono- und Dialkyl-m-dihydroxybenzole in der deutschen Patentschrift Nr. 26 17 739. Resorcin und die bekannten Resorcinderivate befriedigen jedoch nicht in den Echtheitseigenschaften der damit erzielbaren Haarfärbungen. So führt zum Beispiel Resorcin und 2-Methylresorcin in Kombination mit vielen üblichen Entwicklerkomponenten, zum Beispiel mit Tetraaminopyridinen, zu Färbungen, die zur Verrötung neigen, wenn sie dem Licht und der Wärme ausgesetzt sind.

Es wurde nun gefunden, daß Haarfärbemittel, enthaltend Oxidationsfarbstoffvorprodukte in einem kosmetischen Träger, die als Oxidationsfarbstoffvorprodukte Resorcinderivate der allgemeinen Formel I

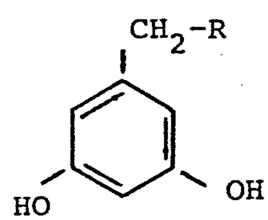

I

in der R eine $-NH_2$-Gruppe, eine -OH-Gruppe oder eine -COOH-Gruppe bedeutet, als Kupplerkomponente und die

...

in Oxidationsfärbemitteln üblichen Entwicklersubstanzen enthalten, die gestellten Anforderungen in hohem Maße erfüllen und darüber hinaus ganz überraschend den Vorteil einer besonderen Echtheit gegen das unerwünschte Verröten unter den Einfluß von Licht und Wärme zeigen. Die erfindungsgemäßen Haarfärbemittel liefern besonders intensive und brillante Haarfärbungen von hoher Lichtechtheit und Wärmestabilität, deren Nuancen vornehmlich im Braunbereich liegen. Die erfindungsgemäß einzusetzenden Resorcinderivate der allgemeinen Formel I sind literaturbekannte Verbindungen.

Als Entwickler können alle hierfür bekannten Verbindungen eingesetzt werden. Besonders wertvolle Farbnuancen werden erhalten, wenn als Entwicklersubstanzen aromatische und/oder heterocyclische Diamine enthalten sind. Solche Entwicklersubstanzen sind zum Beispiel p-Phenylendiamin, p-Toluylendiamin, N-Methyl-p-phenylendiamin, N,N-Dimethyl-p-phenylendiamin, N,N-Diethyl-2-methyl-p-phenylendiamin, N-Ethyl-N-(2-hydroxyethyl)-p-phenylendiamin, Chlor-p-phenylendiamin, N,N-Bis(-2hydroxyethyl)-p-phenylendiamin, Methoxy-p-phenylendiamin, 2.6-Dichlor-p-phenylendiamin, 2-Chlor-6-brom-p-phenylendiamin, 2-Chlor-6-methyl-p-phenylendiamin, 6-Methoxy-3-methyl-p-phenylendiamin, 2.5-Diaminoanisol, N-(2-Hydroxypropyl)-p-phenylendiamin, N-2-Methoxyethyl-p-phenylendiamin und andere Verbindungen der genannten Art, die weiterhin eine oder mehrere $NH_2$-Gruppen, NHR-Gruppen, $NR_2$-Gruppen, wobei R einen Alkylrest mit 1 - 4 oder einen Hydroxyalkylrest mit 2 - 4 Kohlenstoffatomen darstellt, ferner Diaminopyridinderivate, N-Butyl-N-sulfobutyl-p-phenylendiamin sowie besonders Tetraaminopyrimidine wie 2.4.5.6-Tetraaminopyrimidin,

...

4,5-Diamino-2,6-bismethylaminopyrimidin, 2,5-Diamino-4-diethylamino-6-methylaminopyrimidin, 2.4.5-Triamino-6-dimethylaminopyrimidin, 2.4.5-Triamino-6-piperidinopyrimidin, 2.4.5-Triamino-6-anilino-pyrimidin, 2.4.5-Triamino-6-morpholinopyrimidin, 2.4.5-Triamino-6-(2-hydroxyethyl)-aminopyrimidin.

Die erfindungsgemäßen Haarfärbemittel können zusätzlich übliche Kupplersubstanzen, wie zum Beispiel m-Phenylendiaminderivate, Phenole, Naphthole und Pyrazolone enthalten. Auch direktziehende Farbstoffe, zum Beispiel Nitrophenylendiaminderivate, können zur Modifikation der Farbnuancen zugesetzt werden.

In den erfindungsgemäßen Haarfärbemitteln werden die Resorcinderivate und die gegebenenfalls zusätzlich vorhandenen anderen Kupplersubstanzen im allgemeinen in etwa molaren Mengen, bezogen auf die verwendeten Entwicklersubstanzen eingesetzt. Wenn sich auch der molare Einsatz als zweckmäßig erweist, so ist es jedoch nicht nachteilig, einen gewissen Überschuß einzelner Oxidationsfarbstoffvorprodukte zum Einsatz zu bringen.

Es ist auch nicht erforderlich, daß die Resorcinderivate der allgemeinen Formel I sowie die sonst in den Haarfärbemitteln vorhandenen Oxidationsfarbstoffvorprodukte oder direkt ziehenden Farbstoffe einheitliche chemische Verbindungen darstellen. Vielmehr können diese auch Gemische der erfindungsgemäß einzusetzenden Kuppler- oder Entwicklersubstanzen sein.

Die oxidative Entwicklung der Färbung kann grundsätzlich mit Lauftsauerstoff erfolgen. Bevorzugt wird jedoch

...

ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Zur Herstellung der erfindungsgemäßen Haarfärbemittel werden die Oxidationsfarbstoffvorprodukte in einen geeigneten kosmetischen Träger eingearbeitet. Solche Träger sind zum Beispiel Cremes, Emulsionen, Gele oder auch tensidhaltige, schäumende Lösungen, zum Beispiel Shampoos oder andere Zubereitungen, die für die Anwendung auf dem Haar geeignet sind. Übliche Bestandteile solcher kosmetischer Zubereitungen sind zum Beispiel Netz- und Emulgiermittel wie anionische, nichtionische oder ampholytische Tenside, zum Beispiel Fettalkoholsulfate, Alkansulfonate, $\alpha$-Olefinsulfonate, Fettalkoholpolyglycolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel wie zum Beispiel Methyl- oder Hydroxymethylcellulose, Stärke, Fettalkohole, Paraffinöle, Fettsäuren, ferner Parfümöle und haarpflegende Zusätze, wie zum Beispiel wasserlösliche kationische Polymere, Proteinderivate, Pantothensäure und Cholesterin. Die Bestandteile der kosmetischen Träger werden zur Herstellung der erfindungsgemäßen Haarfärbemittel in für diese Zwecke üblichen Mengen eingesetzt; zum Beispiel werden Emulgiermittel in Konzentrationen von 0,5 bis 30 Gewichtsprozent und Verdickungsmittel in Konzentrationen von 0,1 bis 25 Gewichtsprozent des gesamten Färbemittels eingesetzt. Die

...

Oxidationsfarbstoffvorprodukte werden in Mengen von 0,2 bis 5 Gewichtsprozent, vorzugsweise 1 bis 3 Gewichtsprozent des gesamten Färbemittels in den Träger eingemischt.

Die Anwendung der erfindungsgemäßen Haarfärbemittel kann, unabhängig von der Art der kosmetischen Zubereitung, zum Beispiel als Creme, Gel oder Shampoo, im schwach sauren, neutralen oder alkalischen Milieu erfolgen. Bevorzugt ist die Anwendung der Haarfärbemittel in einem pH-Bereich von 8 - 10. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, zum Beispiel ein Färbeshampoo, verwendet wurde.

Die mit den erfindungsgemäßen Haarfärbemitteln erzielbaren Färbungen haben eine hohe Brillanz sowie überlegene Wärme-, Licht-, Wasch- und Reibechtheitseigenschaften. Die nachfolgenden Beispiele sollen den Erfindungsgegenstand näher erläutern, ohne ihn jedoch hierauf zu beschränken.

...

Beispiele

Es wurden erfindungsgemäße Haarfärbemittel in Form einer Haarfärbe-Cremeemulsion der folgenden Zusammensetzung hergestellt.

| | |
|---|---|
| Fettalkohol $C_{12-18}$ | 10 g |
| Fettalkohol $C_{12-14}$ + 2 EO-sulfat, | |
| Na-Salz, 28 %ig | 25 g |
| Wasser | 60 g |
| Resorcinderivat | 0,0075 Mol |
| Entwicklersubstanz | 0,0075 Mol |
| $Na_2SO_3$ (Inhibitor) | 1,0 g |
| konzentrierte Ammoniak-Lösung | bis pH = 9,5 |
| Wasser | ad 100 g |

Die Bestandteile wurden der Reihe nach miteinander vermischt. Nach Zugabe der Oxidationsfärbemittelvorprodukte und des Inhibitors wurde zunächst mit konzentrierter Ammoniak-Lösung der pH-Wert der Emulsion auf 9,5 eingestellt, dann wurde mit Wasser auf 100 g aufgefüllt.

Die oxidative Entwicklung der Färbung wurde mit 3 %iger Wasserstoffperoxidlösung als Oxidationsmittel durchgeführt. Hierzu wurden 100 g der Emulsion mit 50 g Wasserstoffperoxidlösung (3 %ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Als Resorcinderivate wurden die folgenden Verbindungen eingesetzt:

...

K 1: 3,5-Dihydroxybenzylalkohol
Die Herstellung erfolgte nach Th. Boehm und H. Parlasca, Archiv der Pharmazie 270 (1932), S. 175

K 2: 3,5-Dihydroxybenzylamin
Die Herstellung erfolgte nach M. Ikeda et al., Tetrahedron Vol. 33 (1977), S. 491

K 3: 3,5-Dihydroxyphenylessigsäure
Die Herstellung erfolgte nach Theilacker u. Schmid, Annalen der Chemie 570 (1950), S. 27

Als Entwicklersubstanzen wurden die folgenden Verbindungen eingesetzt:

E 1: 2.4.5.6-Tetraaminopyrimidin
E 2: p-Phenylendiamin
E 3: p-Toluylendiamin
E 4: 2.5-Diaminoanisol
E 5: 2-Chlor-o-phenylendiamin
E 6: N-Ethyl-N(2-hydroxyethyl)-p-phenylendiamin
E 7: N-Butyl-N-sulfobutyl-p-phenylendiamin
E 8: N,N-Bis-(2-hydroxyethyl)-p-phenylendiamin
E 9: N-Methyl-p-phenylendiamin
E 10: N-2-Methoxyethyl-p-phenylendiamin
E 11: N-2-Hydroxypropyl-p-phenylendiamin
E 12: p-Aminophenol

Die mit diesen Oxidationsfarbstoffvorprodukten erhaltenen Färbungen sind der Tabelle I zu entnehmen.

...

Tabelle I

| Beispiel | Entwickler | Kuppler | erhaltener Farbton |
|---|---|---|---|
| 1 | E 1 | K 1 | rotbraun |
| 2 | E 2 | K 1 | dunkelviolett |
| 3 | E 3 | K 1 | dunkelbraun |
| 4 | E 4 | K 1 | dunkelmagenta |
| 5 | E 5 | K 1 | mittelbraun |
| 6 | E 6 | K 1 | graurubin |
| 7 | E 7 | K 1 | graubraun |
| 8 | E 8 | K 1 | graubraun |
| 9 | E 9 | K 1 | nutria |
| 10 | E 10 | K 1 | graubraun |
| 11 | E 11 | K 1 | graubraun |
| 12 | E 12 | K 1 | braun |
| 13 | E 1 | K 2 | violettbraun |
| 14 | E 3 | K 2 | dunkelbraun |
| 15 | E 1 | K 3 | orangebraun |
| 16 | E 3 | K 3 | braun |

...

0106089
HENKEL KGaA
ZR-FE/Patente

## Patentansprüche

1. Haarfärbemittel, enthaltend Oxidationsfarbstoffvorprodukte in einem kosmetischen Träger, dadurch gekennzeichnet, daß als Oxidationsfarbstoffvorprodukte Resorcinderivate der allgemeinen Formel

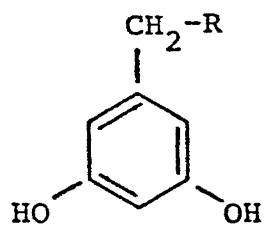

in der R eine $-NH_2$-Gruppe, eine -OH-Gruppe oder eine -COOH-Gruppe bedeutet oder deren Salze als Kupplerkomponente und die in Oxidationsfärbemitteln üblichen Entwicklersubstanzen enthalten sind.

2. Haarfärbemittel nach Anspruch 1, dadurch gekennzeichnet, daß als Entwicklersubstanzen aromatische und/oder heterocyclische Diamine enthalten sind.

3. Haarfärbemittel nach Anspruch 1, dadurch gekennzeichnet, daß als Entwicklersubstanzen 2.4.5.6-Tetraaminopyrimidin und/oder Derivate davon enthalten sind.

4. Haarfärbemittel nach Anspruch 1-3, dadurch gekennzeichnet, daß zusätzlich weitere, bekannte Kupplersubstanzen und/oder direkt ziehende Farbstoffe enthalten sind.

5. Haarfärbemittel nach Anspruch 1-4, dadurch gekennzeichnet, daß der Gehalt an Oxidationsfarbstoffvorprodukten 0,2 bis 5,0 Gewichtsprozent des gesamten Färbemittels ausmacht.

0106089

Europäisches Patentamt

# EUROPÄISCHER RECHERCHENBERICHT

Nummer der Anmeldung

EP 83 10 8618

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| --- | --- | --- | --- |
| Y | FR-A-2 348 696 (HENKEL) * Insgesamt * --- | 1-5 | A 61 K 7/13 |
| D,Y | TETRAHEDRON, Band 33, Nr. 5, 1977, Seiten 489-495, Oxford, GB. M. IKEDA et al.: "Photochemical synthesis of 1,2,3,4-tetrahydroisoquinolin-3-ones from N-chloroacetylbenzylamines" * Seite 491, rechte Spalte, Verbindung 3 * --- | 1-5 | |
| Y | CHEMICAL ABSTRACTS, Band 45, Nr. 13, 10. Juli 1951, Spalten 5672c-5674a, Columbus, Ohio, USA W. THEILACKER et al.: "The structure of triacylmethanes. I. Attempted resolution of asymmetrical triacylmethanes" & ANN. 570, 1-15(1950) * Spalte 5672, Absatz i * --- | 1-5 | RECHERCHIERTE SACHGEBIETE (Int. Cl. 3) A 61 K 7/00 D 06 P 1/00 D 06 P 3/00 |
| Y | "Beilsteins Handbuch der organischen Chemie", 4. Auflage, Band 6, 2. Ergänzungswerk, 1944, Seite 1084, Springer Verlag, Berlin, DE. * Seite 1084, Nr. 9 * --- | 1-5 | |
| A | EP-A-0 026 473 (WELLA) * Ansprüche * ----- | | |

Der vorliegende Recherchenbericht wurde für alle Patentansprüche erstellt.

| Recherchenort | Abschlußdatum der Recherche | Prüfer |
| --- | --- | --- |
| DEN HAAG | 01-02-1984 | BENZ K.F. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument